# EUROPÄISCHE PATENTANMELDUNG

(11) **EP 1 123 693 A2**
(43) Veröffentlichungstag der Anmeldung: **16.08.2001**
(21) Anmeldenummer: 01101900.7
(22) Anmeldetag: 27.01.2001
(51) Int. Cl.: A61K 7/06

(54) **Haarkonditionierungsmittel**

(30) Priorität: 08.02.2000 DE 10005162
(71) Anmelder: Henkel Kommanditgesellschaft auf Aktien, 40589 Düsseldorf-Holthausen (DE)
(72) Erfinder: Höffkes, Horst, Dr., 40595 Düsseldorf (DE); Bergmann, Bettina, 40667 Meerbusch (DE)

(57) **Zusammenfassung**

Durch eine Wirkstoffkombination bestehend aus einer verzweigten, gesättigten Fettsäure, einem Ölkörper sowie mindestens einem Tensid, welches ausgewählt ist aus der Gruppe der Alkylpolyglucoside, der Fettsäure-N-Alkylpolyhydroxyalkylamide, der Amphotenside, der zwitterionischen Tenside oder der Aminoxide, läßt sich die Kämmbarkeit keratinischer Fasern deutlich verbessern.

## Beschreibung

Die Erfindung betrifft kosmetische Mittel mit einer speziellen Wirkstoffkombination sowie Verfahren zur Behandlung von Haaren unter Verwendung dieser Mittel.

Das menschliche Haupthaar wird heute in vielfältiger Weise mit haarkosmetischen Zubereitungen behandelt. Dazu gehört beispielsweise die Reinigung der Haare mit Shampoos und Duschpräparaten und das Bleichen, Färben und Verformen von Haaren mit Wellmitteln, Tönungsmitteln und Stylingpräparaten. Als Folge fast aller Haarbehandlungen kann es zu unerwünschten Beeinträchtigungen der Haarstruktur kommen. Diese Beeinträchtigungen zeigen sich u. a. in einer schlechten Naß- und Trockenkämmbarkeit, einer verstärkten elektrostatischen Aufladung, verstärkter Sprödigkeit, verringerter Höchstreißkraft und Reißdehnung der Haare sowie einem verschlechterten äußeren Erscheinungsbild der Frisur. Weiterhin können beispielsweise Färbemittel in einigen Fällen zu noch mit dem bloßen Auge erkennbar ungleichmäßigen Ausfärbungen führen.

Es hat daher nicht an Versuchen gefehlt, diese Mißstände zu bekämpfen. Dabei wurden u. a. spezielle Nachbehandlungsmittel entwickelt, die insbesondere zur Verbesserung der Struktur und der Eigenschaften geschädigter Haare, das heißt zur Wiederherstellung der physikalischen Eigenschaften gesunder Haare dienen. Weiterhin wurden Wirkstoffkombinationen gefunden, deren Einarbeitung in bekannte Haarbehandlungsmittel den genannten Haarschädigungen in möglichst großem Umfang vorbeugen sollen, oder auf andere Weise das Erscheinungsbild der behandelten Haare verbessern. Als Beispiele für eine solche Verbesserung seien hier egalisierende Bestandteile von Haarfärbemitteln oder restrukturierend wirkende Rohstoffe in Shampoos oder Wellmitteln genannt.

Es besteht aber weiterhin ein Bedarf an haarkosmetischen Mitteln, die sich durch eine Verringerung der unerwünschten Beeinträchtigungen der Haare auszeichnen. Es wurde nunmehr gefunden, daß die Verwendung einer bestimmten Wirkstoffkombination in kosmetischen Mitteln zu überraschend guten Eigenschaften der behandelten Haare, insbesondere zu einer verbesserten Naßkämmbarkeit, führt.

Gegenstand der Erfindung ist ein kosmetisches Mittel enthaltend übliche kosmetische Bestandteile, dadurch gekennzeichnet, daß es eine Wirkstoffkombination bestehend aus:
a) 0,1 - 15 Gew.% mindestens einer verzweigten, gesättigten Fettsäure (A) mit 6 - 30 Kohlenstoffatomen
b) 0,1 - 15 Gew.% mindestens eines Ölkörpers (B) mit einem Schmelzpunkt von kleiner 55°C
c) 0,1 - 20 Gew.% mindestens eines Tensides (C) ausgewählt aus der Gruppe der Alkylpolyglykoside, der Fettsäure-N-Alkylpolyhydroxyalkylamide, der amphoteren Tenside, der zwitterionischen Tenside oder der Aminoxide
enthält.

Die Verwendung von Fettsäuren in Färbe- und Bleichmitteln ist bekannt aus den Druckschriften EP 0 763 355 A1, EP 0 938 888 A2 sowie WO 94/04125 A1. In der deutschen Anmeldung mit dem Aktenzeichen P 198 35 327.8 wird die Kombination aus Fettalkohol und Isostearinsäure offenbart. Es ist in diesen Schriften jedoch keinerlei Hinweis auf eine synergistische Wirkung der erfindungsgemäßen Wirkstoffkombination aus den Komponenten (A), (B) und (C) zu entnehmen.

Die erfindungsgemäße Komponente (A) ist eine verzweigte, gesättigte Fettsäure mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95. Die Einsatzmenge beträgt dabei 0,1 - 15 Gew.%, bezogen auf das gesamte Mittel.

In einer bevorzugten Ausführungsform beträgt die Menge 0,5 - 10 Gew.%, wobei ganz besonders vorteilhaft Mengen von 1 - 5 Gew.% sind.

Unter der erfindungsgemäßen Verbindung (B) ist mindestens ein Ölkörper mit einem Schmelzbereich unterhalb von 55°C zu verstehen. Zu den erfindungsgemäßen, bei Raumtemperatur festen Ölkörpern sind zu zählen:
a) gesättigte, ein- oder mehrfach ungesättigte, verzweigte oder unverzweigte Fettalkohole mit C6 - C30-, bevorzugt C10 - C22- und ganz besonders bevorzugt C12 - C18- Kohlenstoffatomen. Solche Substanzen sind beispielsweise unter den Bezeichnungen Stenol® , z. B. Stenol® 1618 oder Lanette® , z. B. Lanette® O oder Lorol® , z. B. Lorol® C8, Lorol® C14, Lorol® C18, Lorol® C8-18, HD-Ocenol® , Hydrenol® D, Crodacol® , z. B. Crodacol® CS oder Novol® käuflich zu erwerben.
b) Wollwachsalkohole wie sie beispielsweise unter den Bezeichnungen Corona®, White Swan® , Coronet® oder Fluilan® käuflich zu erwerben sind.
c) Feste Paraffine oder Isoparaffine
Die erfindungsgemäßen flüssigen Ölkörpern (B) sind:
a) Guerbetalkohole wie z. B. Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24.
b) Flüssige Paraffine oder Isoparaffine
c) Esteröle. Unter Esterölen sind zu verstehen die Ester von C6 - C30 - Fettsäuren mit C2 - C30 - Alkoholen, wie beispielsweise Oleylerucat (Handelsprodukt Cetiol® J 600), Isopropylmyristat (Handelsprodukt Rilanit® IPM), Isopropylpalmitat (Handelsprodukt Rilanit® IPP) und Decyloleate (Handelsprodukt Cetiol® V).
d) Symmetrische oder unsymmetrische Dialkylether mit 1 - 36 Kohlenstoffatomen je Alkylrest, wobei die Summe der Kohlenstoffatome mindestens 8 ist, wie das Handelsprodukt Cetiol® OE.
Die Einsatzmenge beträgt 0,1 - 15 Gew.% bezogen auf das gesamte Mittel, bevorzugt 0,5 - 10 Gew.% und besonders bevorzugt 1 - 5 Gew.% bezogen auf das gesamte Mittel. Schließlich enthalten die erfindungsgemäßen Mittel mindestens ein Tensid (C), ausgewählt aus der Gruppe der Alkylpolyglykoside, der Fettsäure-N-Alkylpolyhydroxyalkylamide, der amphoteren Tenside, der zwitterionischen Tenside oder der Aminoxide.
Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat, das als Dehyton® K erhältlich ist.

Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Die Alkylpolyglykoside entsprechen der allgemeinen Formel RO-(Z)ₓ wobei R für C₆₋₃₀ Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt. Solche Verbindungen sind beispielsweise unter dem Markenzeichen Plantacare® erhältlich.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Bevorzugte Tenside (C) sind zwitterionische und amphotere Tenside. Die Tenside (C) werden in Mengen von 0,1 - 45 Gew.%, bevorzugt 1 - 30 Gew.% und ganz besonders bevorzugt von 1 - 15 Gew.%, bezogen auf das gesamte Mittel, eingesetzt.

Ein als Tensid (C) bevorzugtes Aminoxid ist das Handelsprodukt Aminoxid® WS35. Als Fettsäure-N-Alkylpolyhydroxyalkylamide sind erfindungsgemäß insbesondere die in der DE-P4430085.9-41, auf die ausdrücklich Bezug genommen wird, offenbarten Verbindungen verwendbar.

Gemäß einer bevorzugten Ausführungsform enthalten die erfindungsgemäßen Mittel weiterhin ein Aniontensid. Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist, wie die Produkte Texapon® N70 und Texapon® NSO.
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkylphosphate und Alkylpolyglykoletherphosphate der Formel [R-O(CH₂-CH₂O)ₓ-O]_{y}PO_{z}H, in der R eine bevorzugt lineare Alkylgruppe mit 10 bis 18 C-Atomen, x = 0 oder 1 bis 12 ist y = 1 bis 3 und z = 1 - 3 sind, wobei die Summe y + z = 4 ist.
- Eiweißfettsäurekondensate, wie beispielsweise Gluadin® WK.
- Acylglutamate, wie beispielsweise Hostapon® KCG.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Acyltauride mit 10 bis 18 C-Atomen in der Acylgruppe, Alkylphosphate und Alkylpolyglykoletherphosphate, wie zuvor beschrieben, Eiweißfettsäurekondensate sowie Acylglutamate.

Weiterhin kann es bevorzugt sein, in den erfindungsgemäßen Haarbehandlungsmitteln - kationischen Tenside einzusetzen. Beispiele hierfür sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Alkylamidoamine, insbesondere Fettsäureamidoamine wie das unter der Bezeichnung Tego Amid® S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die Produkte Dehyquart® AU-46, Dehyquart® F-30, Dehyquart® C4046, Dehyquart® L80 und Dehyquart® F-75 sowie die unter dem Warenzeichen Stepantex® vertriebenen Dialkylammoniummethosulfate und Methyl-hydroxyalkyl-dialkoyloxyalkylammoniummethosulfate.

Weitere geeignete erfindungsgemäße kationische Tenside sind kationische Proteinderivate. Eine Reihe solcher quartärer Proteinhydrolysate sind als Handelsprodukte erhältlich, beispielsweise:
- kationisches Kollagenhydrolysat, beispielsweise das unter der Bezeichnung Lamequat® L auf dem Markt befindliche Produkt (INCI-Bezeichnung: Lauryldimonium Hydroxypropyl Hydrolyzed Collagen; Chemische Fabrik Grünau) mit der Struktur 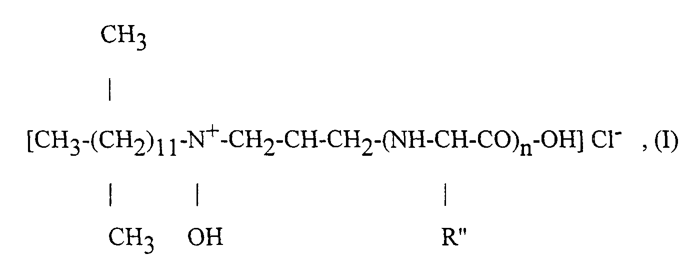 in der R" für die Seitenketten der Aminosäuren des Kollagens steht.
- kationisches Keratinhydrolysat, beispielsweise das unter der Bezeichnung Croquat® auf dem Markt befindliche Produkt (CTFA-Bezeichnung: Cocodimonium Hydroxypropyl Hydrolyzed Keratin; Croda)
- kationisches Weizenhydrolysat, erhältlich unter der Bezeichnung Hydrotriticum® QL (CTFA-Bezeichnung: Lauryldimonium Hydroxypropyl Hydrolyzed Wheat Protein; Croda)
- das unter der Bezeichnung Crotein® Q erhältliche Produkt, gemäß INCI ein "Hydroxypropyltrimonium Hydrolyzed Collagen" (Croda) sowie
- das als Lexein® Q X 3000 (Inolex) erhältliche quaternierte Eiweißhydrolysat.

Ebenfalls bevorzugte weitere Komponenten sind 0,05 - 10 Gew.% , bevorzugt 0,1 - 5 Gew und ganz besonders bevorzugt 0,1 - 3 Gew.%, bezogen auf das gesamte Mittel, eines synthetischen oder natürlichen Polymers.

Gemäß einer ersten Variante dieser Ausführungsform wird erfindungsgemäß zusätzlich ein kationisches Polymer verwendet. Die verwendbaren kationischen Polymeren enthalten innerhalb des Polymergerüstes kationische Gruppen. Diese Gruppen können Teil der Polymerkette sein; sie können sich aber auch in Seitenketten befinden, die über Zwischenglieder mit einer Hauptkette verbunden sind. Übliche kationische Gruppen enthalten quartäre Stickstoff- oder Phosphoratome. Gruppen mit quartären Stickstoffatomen sind dabei bevorzugt. Die quartären Stickstoffatome können dabei sowohl 4 unterschiedliche oder z. T. gleiche Substituenten tragen, als auch Teil eines Ringsystems sein. Bevorzugte kationische Gruppen sind Ammonium- und Imidazoliniumgruppen.

Eine Reihe von für die Haarpflege geeigneten kationischen Polymeren sind dem Fachmann bekannt und als Handelsprodukte erhältlich.

Beispiele für solche Polymeren sind:
- quaternierte Cellulose-Derivate, wie sie unter den Bezeichnungen Celquat® und Polymer JR® im Handel erhältlich sind. Die Verbindungen Celquat H 100, Celquat L 200 und Polymer JR® 400 sind bevorzugte quaternierte Cellulose-Derivate.
- quaternierte Guar-Derivate, wie sie unter den Bezeichnungen Cosmedia Guar® und Jaguar® im Handel erhältlich sind. Bevorzugte Guar-Derivate sind beispielsweise Cosmedia Guar® C-261 und Jaguar® C 13-S.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere sowie das Vinylpyrrolidon-Methacrylamidopropyltrimethylammoniumchlorid-Copolymere. Solche Verbindungen sind unter den Bezeichnungen Gafquat® 734, Gafquat® 755 bzw. Gafquat® HS100 im Handel erhältlich.
- Copolymerisate des Vinylpyrrolidons mit Vinylimidazoliummethochlorid, wie sie unter der Bezeichnung Luviquat® angeboten werden.
- Polymere Dimethyldiallylammoniumsalze und deren Copolymere mit Estern und Amiden von Acrylsäure und Methacrylsäure. Die unter den Bezeichnungen Merquat® 100 (Poly(dimethyldiallylammoniumchlorid)) und Merquat® 550 (Dimethyldiallylammoniumchlorid-Acrylamid-Copolymer) im Handel erhältlichen Produkte sind Beispiele für solche kationischen Polymere.
- Kationisch derivatisierte Silikonöle, wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil® -Quat 3270 und 7232 (Hersteller: Th. Goldschmidt; diquaternäre Polydimethylsiloxane, Quaternium-80).
- Chitosan und dessen Derivate.

Die eingesetzten Mengen betragen 0,05 - 10 Gew.%, bevorzugt 0,1 - 5 und ganz besonders bevorzugt 0,1 - 3 Gew.%, bezogen auf das gesamte Mittel.

Gemäß einer zweiten Variante wird erfindungsgemäß zusätzlich ein anionisches Polymer verwendet. Geeignete anionische Polymere sind beispielsweise:
- Copolymere der Acrylsäure und/oder Methacrylsäure oder deren Ester mit C₁₀₋₃₀-Alkylacrylaten, wie sie beispielsweise unter der Bezeichnung Pemulen® vertrieben werden,
- Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere. Verbindungen dieser Art sind unter den Markenbezeichnungen Resyn® (NATIONAL STARCH), Luviset® (BASF) und Gafset® (GAF) im Handel; die Produkte Luviset® CA-66 und Luviset® CAP können bevorzugt sein,
- Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymere,
- Acrylsäure/Ethylacrylat/N-tert.Butylacrylamid-Terpolymere, die beispielsweise unter der Bezeichnung Ultrahold® strong (BASF) vertrieben werden, sowie Methacrylsäure/Ethylacrylat/t-Butylacrylat-Terpolymer, die unter der Bezeichnung Luvimer® 100P (BASF) vertrieben werden.

Bevorzugt sind letztgenannte Wirkstoffe in Mengen von 0,1 - 5 Gew.-%, bezogen auf das gesamte Mittel, enthalten.

Die erfindungsgemäßen Mittel können in einer dritten Variante weiterhin nichtionogene Polymere enthalten. Die nichtionogenen Polymere können dabei sowohl als Verdicker als auch zur Konditionierung, wie beispielsweise Silikonöle, enthalten sein.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden.
- Phospholipide, beispielsweise Sojalecithin, Ei-Lecithin und Kephaline als Wirkstoffe, insbesondere zur Konditionierung
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.

Die erfindungsgemäßen Mittel können in einer vierten Variante weiterhin amphotere oder zwitterionische Polymere enthalten. Unter "zwitterionischen Polymeren" werden solche Polymeren verstanden, die im Molekül quartäre Ammoniumgruppen und -COO⁻- oder-SO₃⁻-Gruppen enthalten.

Beispiele für erfindungsgemäß einsetzbare zwitterionische Polymere sind die in der britischen Offenlegungsschrift 2 104 091, der europäischen Offenlegungsschrift 47 714, der europäischen Offenlegungsschrift 283 817 und der deutschen Offenlegungsschrift 28 17 369 genannten Verbindungen.

Besonders bevorzugt werden zwitterionische Polymere, die sich im wesentlichen zusammensetzen aus
(α) Monomeren mit quartären Ammoniumgruppen der allgemeinen Formel (II),

   R¹-CH=CR²-CO-X-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, X eine NH-Gruppe oder ein Sauerstoffatom, n eine ganze Zahl von 2 bis 5 und A⁽⁻⁾ das Anion einer organischen oder anorganischen Säure ist
   und
(β) monomeren Carbonsäuren der allgemeinen Formel (III),

   R⁶-CH=CR⁷-COOH (III)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen.
Ganz besonders bevorzugt sind solche Polymeren auf Basis von Monomeren des Typs (α), bei denen R³, R⁴ und R⁵ Methylgruppen sind, X eine NH-Gruppe und A(-) ein Halogenid-, Methoxysulfat- oder Ethoxysulfat-Ion ist; Acrylamidopropyltrimethylammoniumchlorid ist ein besonders bevorzugtes Monomeres (α). Als Monomeres (β) für die genannten Polymeren wird bevorzugt Acrylsäure oder ein Alkalisalz der Acrylsäure, insbesondere das Natriumsalz, verwendet.

Weiterhin sind solche zwitterionischen Polymere bevorzugt, bei denen die Zahl der Monomeren vom Typ (α) größer als die Zahl der Monomeren vom Typ (β) ist. Momomerenverhältnisse (α):(β) größer als 1,5 sind besonders bevorzugt.

In einer weiteren bevorzugten Verwendung enthalten die erfindungsgemäßen Mittel 0,001 - 20 Gew.% an Oxidationsfarbstoffvorprodukten insbesondere sogenannte Entwicklersubstanzen und Kuppler. Weiterhin können in dieser bevorzugten Verwendung alternativ zu oder in Kombination mit den Oxidationsfarbstoffvorprodukten auch direktziehende Farbstoffe enthalten sein.
Als Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 bzw. WO 94/08970 wie z. B. 4,5-Diamino-1-(2'-hydroxyethyl)-pyrazol zu nennen.

Als Kupplerkomponenten werden in der Regel
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2' -Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.
Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere bevorzugte Farbstoffkomponenten sind Isatin und dessen Derivate, wie sie beispielsweise in den deutschen Offenlegungsschriften DE 4211450, DE 4301818 und DE 4314317 offenbart sind.

In einer bevorzugten Ausführung der Erfindung zum Färben von Haar wird die Zubereitung auf einen pH-Wert von 3,0 bis 11,5 eingestellt. Dabei kann die oxidative Entwicklung der Färbung grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen insbesondere Wasserstoffperoxid oder dessen Anlagerungsprodukte an Harnstoff, Melamin oder Natriumborat in Frage. Weiterhin ist es möglich, die Oxidation mit Hilfe von Enzymen durchzuführen. Dabei können die Enzyme sowohl zur Erzeugung von oxidierenden Per-Verbindungen eingesetzt werden, als auch zu Verstärkung der Wirkung einer geringen Mengen vorhandener Oxidationsmittel. Beispiele für enzymatische Verfahren sind das Vorgehen, die Wirkung geringer Mengen (z. B. 1 % und weniger, bezogen auf das gesamte Mittel) Wasserstoffperoxid durch Peroxidasen zu verstärken, oder der Verwendung von Laccasen, Uricasen und anderen zur Haarfärbung eingesetzten Enzymen oder in Kombination mit deren Substraten.

Zweckmäßigerweise wird die Zubereitung des Oxidationsmittels unmittelbar vor dem Färben der Haare mit der Zubereitung mit den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Die weiteren Bestandteile der erfindungsgemäßen Haarbehandlungsmittel sind von der Art des Haarbehandlungsmittels abhängig. Dabei kann die erfindungsgemäße Wirkstoffkombination ein wesentliches Wirkprinzip des speziellen Haarbehandlungsmittels sein; die Wirkstoffkombination kann aber auch in Mittel eingearbeitet werden, die primär einem anderen Zweck, beispielsweise der Reinigung oder der Färbung dienen.

Als weitere mögliche Bestandteile sind nichtionische Tenside, welche nicht unter die Definition des Tensides (C) fallen, zu nennen. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare und verzweigte Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Als bevorzugte nichtionische Tenside haben sich die Alkylenoxid-Anlagerungsprodukte an gesättigte lineare Fettalkohole und Fettsäuren mit jeweils 4 bis 20 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Bestandteile der erfindungsgemäßen Mittel können beispielsweise sein:
- Strukturanten wie Maleinsäure,
- Verdickungsmittel wie Agar-Agar, Guar-Gum, Alginate und Xanthan-Gum,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine, Zink Omadine, Ketoconazol, Eluidin und Climbazol,
- weitere Substanzen zur Einstellung des pH-Wertes,
- Wirkstoffe wie Allantoin, Pyrrolidoncarbonsäuren und deren Salze, Pflanzenextrakte und Vitamine, insbesondere Panthenol und Vitamin E, wie das Handelsprodukt Copherol® F1300
- Lichtschutzmittel,
- Konsistenzgeber wie Polyolester oder Polyolalkylether,
- Fette und Wachse, wie Walrat, Bienenwachs, Montanwachs,
- Fettsäurealkanolamide,
- Komplexbildner wie EDTA, EDETA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien,
- Reduktionsmittel wie z. B. Thioglykolsäure und deren Derivate, Thiomilchsäure, Cysteamin, Thioäpfelsäure und ∝-Mercaptoethansulfonsäure,
- Oxidationsmittel wie Wasserstoffperoxid, Kaliumbromat und Natriumbromat.

Prinzipiell umfassen die erfindungsgemäßen Formulierungen aber alle bekannten Arten von Haarbehandlungsmitteln wie z. B. Haarshampoos, Haarspülungen, Haarkonditioniermittel, Haarkuren, Haarfestiger, Haarsprays, Fönwellen, Dauerwellmittel und Haarfärbemittel. Haarkuren und Haarkonditioniermittel, insbesondere solche Mittel, die morgens auf das Haar aufgetragen werden, um diesen für den weiteren Verlauf des Tages eine bestimmte Festigung zu geben, sowie Haarshampoos stellen bevorzugte Formen der erfindungsgemäßen Mittel dar.

Die erfindungsgemäß verwendbaren Mittel können als Lösung, Lotion, Emulsion, Mikroemulsion, Creme, Gel oder Schaumaerosol formuliert sein. Die Formulierung als Lösung, Dispersion, Emulsion oder Mikroemulsion mit einem Wassergehalt von 50 bis 90 Gew.-%, bezogen auf das gesamte Mittel, kann bevorzugt sein.

Gegenstand der Erfindung ist auch die Verwendung der erfindungsgemäßen Mittel zur Behandlung von Keratinfasern, insbesondere von Haaren.

Gegenstand der Erfindung ist schließlich auch ein Verfahren zur Behandlung von Haaren, bei dem eine erfindungsgemäße Zubereitung auf das Haar aufgebracht und nach einer Einwirkzeit (0,5 bis 40 Minuten) wieder ausgespült wird.

Gegenstand der Erfindung ist ebenfalls ein Verfahren zur Behandlung von Haaren, bei dem eine erfindungsgemäße Zubereitung auf das Haar aufgebracht wird und dort verbleibt.

Die folgenden Beispiele sollen die Erfindung näher erläutern.

### Beispiele

Alle Angaben sind, soweit nicht anders vermerkt, Gewichtsteile.

### 1. Färbemittel

Rezepturen von erfindungsgemäßen Färbemitteln sind in Tabelle 1 aufgeführt.

**Tabelle 1**

| Rezeptur-Nr. | 1 | 2 | 3 | 4 | 5 | 6 | 7 | 8 | 9 |
|---|---|---|---|---|---|---|---|---|---|
| Hydrenol® D | 7,5 | 8,0 | 8,2 | 9,0 | 8,2 | 8,2 | 8,2 | 8,2 | 8,5 |
| Lorol® C12-C18 | 2,8 | 2,3 | 2,1 | 1,3 | 2,1 | 2,1 | 2,1 | 2,1 | 1,8 |
| Texapon® NSO | 14,0 | 12,0 | 16,0 | 16,0 | 16,0 | 16,0 | 16,0 | 16,0 | 16,0 |
| Dehyton® K | 12,0 | 14,0 | 10,0 | 8,0 | 8,0 | 9,0 | 5,0 | 8,0 | 4,0 |
| Ölsäure | 0,5 | ---- | 2,0 | ---- | ---- | ---- | ---- | ---- | ---- |
| Isostearinsäure | 8,0 | 6,0 | 6,0 | 5,5 | 7,0 | 3,0 | 5,0 | 3,0 | ---- |
| Laurinsäure | ---- | 1,0 | ---- | 2,5 | 1,0 | ---- | ---- | ---- | ---- |
| Isopalmitinsäure | ---- | 2,0 | ---- | ---- | ---- | ---- | ---- | ---- | 3,0 |
| Myristinsäure | ---- | ---- | ---- | ---- | ---- | 5,0 | ---- | ---- | ---- |
| Plantacare® 2000 UP | ---- | ---- | ---- | 2,0 | 2,0 | 1,0 | 5,0 | ---- | ---- |
| Gluadin® WK | ---- | ---- | ---- | ---- | ---- | ---- | ---- | 2,0 | ---- |
| Aminoxid® WS 35 | ---- | ---- | ---- | ---- | ---- | ---- | ---- | ---- | 4,0 |
| Westvaco Diacid® H240 | ---- | ---- | ---- | ---- | ---- | ---- | 3,0 | 5,0 | |
| Polymer® JR 400 | ---- | ---- | ---- | ---- | ---- | 0,4 | ---- | 0,4 | 1,0 |
| Polymer P1, gemäß DE 39 29 973 | ---- | ---- | ---- | ---- | 5,0 | 2,0 | 3,0 | ---- | ---- |
| 1,2-Propylenglycol | 2,0 | 2,0 | 2,0 | 2,0 | 2,0 | 2,0 | 2,0 | 2,0 | 2,0 |
| 5-Amino-2-methylphenol | 0,6 | ---- | 0,6 | 0,6 | 0,6 | ---- | 0,6 | 0,6 | 0,6 |
| 3-Methyl-4-aminophenol | 0,06 | 0,06 | 0,06 | 0,06 | 0,06 | 0,06 | 0,06 | 0,06 | 0,06 |
| 2-Amino-2-hydroxypyrimidin | ---- | 0,55 | ---- | ---- | ---- | 0,55 | ---- | ---- | ---- |
| p-Aminophenolhydrochlorid | 0,6 | 0,6 | 0,6 | 0,6 | 0,6 | 0,6 | 0,6 | 0,6 | 0,6 |
| p-Toluylendiaminsulfat | 0,7 | ---- | 0,7 | 0,7 | 0,7 | ---- | 0,7 | 0,7 | 0,7 |
| 1-(2'-Hydroxyethyl)-2,5-diaminobenzol | ---- | 0,85 | ---- | ---- | ---- | 0,85 | ---- | ---- | ---- |
| Resorcin | 0,16 | 0,16 | 0,16 | 0,16 | 0,16 | 0,16 | 0,16 | 0,16 | 0,16 |
| Ammoniumchlorid | 0,1 | 0,1 | 0,1 | 0,1 | 0,1 | 0,1 | 0,1 | 0,1 | 0,1 |
| Natriumsulfit | 0,5 | 0,5 | 0,5 | 0,5 | 0,5 | 0,5 | 0,5 | 0,5 | 0,5 |
| Komplexierungsmittel | 0,1 | 0,1 | 0,1 | 0,1 | 0,1 | 0,1 | 0,1 | 0,1 | 0,1 |
| Parfümöl | 0,2 | 0,2 | 0,2 | 0,2 | 0,2 | 0,2 | 0,2 | 0,2 | 0,2 |
| Wasser | Ad 100 | | | | | | | | |

Die Mittel wurden zuerst mit KOH auf einen pH-Wert von 8 und dann mit Ammoniak auf einen pH-Wert von 10 eingestellt.

### 2. Shampoo

| | |
|---|---|
| Plantacare® 818 UP | 4,0 |
| Texapon® N70 | 10,0 |
| Dehyton® K | 4,0 |
| Copherol® F 1300 | 0,2 |
| Gluadin® W 40 | 2,0 |
| Isostearinsäure | 2,0 |
| Lanette® O | 0,5 |
| Cosmedia® Guar C 261 | 0,3 |
| Natriumchlorid | 1,5 |
| Citronensäure | bis pH 4,7 - 5,2 |
| Konservierungsmittel | 0,3 |
| Wasser | ad 100 |

### 3. Haarspülung

| | |
|---|---|
| Dehyquart® F 75 | 1,5 |
| Lanette® O | 2,4 |
| Isostearinsäure | 0,8 |
| Plantacare® 1200 UP | 2,0 |
| Cetiol® OE | 1,0 |
| Gluadin® WQ | 2,5 |
| Panthenol | 0,5 |
| Generol® 122 | 0,4 |
| Konservierungsmittel | 0,3 |
| Wasser | ad 100 |

## Patentansprüche

1. Kosmetisches Mittel, enthaltend übliche kosmetische Rohstoffe, dadurch gekennzeichnet, daß es eine Wirkstoffkombination bestehend aus:
a) 0,1 - 15 Gew.% mindestens einer verzweigten, gesättigten Fettsäure (A) mit 6 - 30 Kohlenstoffatomen,
b) 0,1 - 15 Gew.% mindestens eines Ölkörpers (B) mit einem Schmelzbereich kleiner 55°C,
c) 0,1 - 20 Gew.% mindestens eines Tensides (C) ausgewählt aus der Gruppe der Alkylpolyglykoside, der Fettsäure-N-Alkylpolyhydroxyalkylamide, der amphoteren Tenside, der zwitterionischen Tenside oder der Aminoxide
enthält.

2. Kosmetisches Mittel nach Anspruch 1, dadurch gekennzeichnet, daß die Fettsäure (A) 10 - 22 Kohlenstoffatome enthält.

3. Kosmetisches Mittel nach Anspruch 1 oder 2, dadurch gekennzeichnet, daß die unterhalb 55°C schmelzenden Ölkörper Fettalkohole mit 6 - 22 Kohlenstoffatomen, die verzweigt, unverzweigt, gesättigt, ungesättigt sind oder Wollwachsalkohole oder Mischungen daraus sind.

4. Kosmetisches Mittel nach einem der Ansprüche 1 oder 2, dadurch gekennzeichnet, daß die Ölkörper (B) ausgewählt sind aus der Gruppe der Guerbetalkohole, der symmetrischen oder unsymmetrischen Dialkylether mit 1 - 36 Kohlenstoffatomen je Alkylrest, wobei die Summe der Kohlenstoffatome mindestens 8 ist, der Esteröle oder der verzweigten oder unverzweigten Paraffinöle.

5. Kosmetisches Mittel nach einem der Ansprüche 1 bis 4, dadurch gekennzeichnet, daß das Tensid (C) zwitterionisch oder amphoter ist.

6. Kosmetisches Mittel nach einem der Ansprüch 1 bis 5, dadurch gekennzeichnet, daß als weiterer kosmetischer Rohstoff 0,1 - 45 Gew.% mindestens eines anionischen Tensides, ausgewählt aus der Gruppe
- lineare und verzweigte Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acyltauride mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Alkylphosphate und Alkylpolyglykoletherphosphate der Formel [R-O (CH₂-CH₂O)ₓ-O]_{y}PO_{z}H, in der R eine bevorzugt lineare Alkylgruppe mit 10 bis 18 C-Atomen, x = 0 oder 1 bis 12 ist y = 1 bis 3 und z = 1 - 3 sind, wobei die Summe y + z = 4 ist.
- der Eiweißfettsäurekondensate,
- der Acylglutamate,
enthalten ist.

7. Kosmetisches Mittel nach einem der Ansprüche 1 bis 6, dadurch gekennzeichnet, daß als weitere Komponente 0,05 - 10 Gew.% kationische Tenside und/oder natürliche oder synthetische Polymere enthalten sind.

8. Kosmetisches Mittel nach einem der Ansprüche 1 bis 7, dadurch gekennzeichnet, daß als weiterer Bestandteil 0,001 - 20 Gew.% Oxidationsfarbstoffvorprodukte und/oder direktziehende Farbstoffe enthalten sind.

9. Verwendung eines kosmetischen Mittels nach einem der Ansprüche 1 bis 8 zur Behandlung keratinischer Fasern, insbesondere menschlichem Haar.

10. Verfahren zur Behandlung von Haaren, dadurch gekennzeichnet, daß ein kosmetisches Mittel nach einem der Ansprüche 1 bis 8 auf das Haar aufgebracht wird und nach einer Einwirkzeit von 0,5 - 40 Minuten wieder ausgespült wird.
